**Europäisches Patentamt**

(19) **European Patent Office**

**Office européen des brevets**

(11) **EP 0 552 341 B1**

(12) **FASCICULE DE BREVET EUROPEEN**

(45) Date de publication et mention
de la délivrance du brevet:
**16.12.1998 Bulletin 1998/51**

(21) Numéro de dépôt: **92916843.3**

(22) Date de dépôt: **23.07.1992**

(51) Int. Cl.$^6$: **A61N 5/02**

(86) Numéro de dépôt international:
**PCT/FR92/00729**

(87) Numéro de publication internationale:
**WO 93/02747 (18.02.1993 Gazette 1993/05)**

(54) **SYSTEME POUR LE TRAITEMENT HYPERTHERMIQUE D'UN CORPS ET PROCEDE DE CALIBRATION**

SYSTEM ZUR HYPERTHERMIEBEHANDLUNG EINES KÖRPERS UND VERFAHREN ZUR KALIBRIERUNG

SYSTEM FOR HYPERTHERMIA TREATMENT OF A BODY AND METHOD FOR CALIBRATION

(84) Etats contractants désignés:
**CH DE GB IT LI SE**

(30) Priorité: **26.07.1991 FR 9109521**

(43) Date de publication de la demande:
**28.07.1993 Bulletin 1993/30**

(73) Titulaires:
- **INSTITUT NATIONAL DE LA SANTE ET DE LA RECHERCHE MEDICALE (INSERM)**
  **75654 Paris Cédex 13 (FR)**
- **UNIVERSITE DES SCIENCES ET TECHNOLOGIES DE LILLE**
  **F-59655 Villeneuve-d'Ascq (FR)**

(72) Inventeurs:
- **CHIVE, Maurice**
  **F-59650 Villeneuve-d'Ascq (FR)**
- **SOZANSKI, Jean-Pierre**
  **F-59239 Thumeries (FR)**

- **MOSCHETTO, Yves**
  **6, rue du Quesne**
  **F-59230 Haubourdin (FR)**
- **VANLOOT, Daniel**
  **F-62100 Calais (FR)**

(74) Mandataire: **Nuss, Pierre et al**
  **10, rue Jacques Kablé**
  **67080 Strasbourg Cédex (FR)**

(56) Documents cités:
**EP-A- 0 370 890          WO-A-88/03823**
**FR-A- 2 650 390**

- **DATABASE WPIL Week 8819, Derwent Publications Ltd., London, GB; AN 88-132135 & SU,A,1 345 142 (IOSELSON) 15 Octobre 1987**
- **DATABASE WPIL Week 9103, Derwent Publications Ltd., London, GB; AN 91-020869 & SU,A,1 567 203 (KOLESHKO) 30 Mai 1990**

**Description**

L'invention est relative à un système pour le traitement thermique interne d'un corps certain et à l'utilisation de ce système.

Les appareils de traitement thermique interne sont normalement utilisés soit dans les processus industriels soit, selon une tendance nouvelle, dans les méthodes de traitement thérapeutique par hyperthermie.

Dans les deux cas précités, un élément essentiel de ces types d'appareillage est la précision tant de la température que du domaine d'application, par rapport à la zone à traiter, de l'hyperthermie engendrée.

Lorsque de tels modes opératoires sont mis en oeuvre notamment en vue du traitement thermique d'un corps certain, tel que le corps humain par exemple, une méthode consiste à introduire dans une cavité naturelle de celui-ci un élément émetteur et/ou concentrateur d'une énergie, telle qu'une énergie électromagnétique micro-onde, le caractère dissipatif des parois de cette cavité, sièges d'un champ électrique résultant étant alors soumis à une élévation correspondante de température.

Un tel mode opératoire, bien que susceptible de donner satisfaction, ne permet d'établir avec un degré de précision suffisant, ni la température effectivement atteinte par la zone à traiter, ni le domaine du corps certain et donc de la zone à traiter dans ce domaine soumis à l'hyperthermie engendrée, sauf à prévoir un ou plusieurs capteurs de température disposés au voisinage de la zone précitée.

Dans le cas où le corps certain soumis au traitement est le corps humain, il va de soi que l'utilisation d'une cavité naturelle afin de réaliser l'implantation de capteurs de température précités peut être envisagée. Mais un tel mode opératoire présente alors l'inconvénient d'occuper la cavité naturelle correspondante, ce qui pratiquement interdit l'utilisation de celle-ci pour l'implantation d'une source ou d'un élément concentrateur d'énergie génératrice d'hyperthermie. Dans un tel cas, le caractère de proximité de deux cavités naturelles voisines ne peut être utilisé pour provoquer par exemple le traitement par hyperthermie d'une zone à traiter située entre celles-ci.

En outre, on notera que l'utilisation de capteurs de température placés au voisinage de l'élément source d'énergie électromagnétique engendrant l'hyperthermie peut être néfaste pour le fonctionnement de ces capteurs, notamment en raison du fait que les éléments semi-conducteurs actifs de ces derniers sont susceptibles d'être soumis, au cours du fonctionnement, à un champ électrique intense.

Um tel systéme selon l'invention présente notamment les caractéristiques mentionées dans la revendication 1. On connait, par ailleurs, du document FR-A-2650 390 la mise en oeuvre d'un système pour le traitement thermique interne de corps certains, par application d'énergie micro-onde, dans lequel un ou plusieurs applicateurs d'énergie micro-onde sont prévus, ce ou ces applicateurs étant, outre leur fonction d'applicateur, destinés à jouer le rôle de capteur de température en l'absence temporaire d'application d'énergie micro-onde, ce qui permet d'effectuer une mesure de la température instantanée en des points distincts au voisinage de la zone à traiter, ces points correspondant exactement aux points d'application de l'énergie micro-onde au voisinage de la zone à traiter.

La présente invention a pour objet de remédier à l'ensemble des inconvénients précités par la mise en oeuvre d'un système pour le traitement thermique interne de corps certains, par application d'énergie micro-onde, permettant d'obtenir une précision satisfaisante tant de la température atteinte que du domaine dans lequel l'hyperthermie est engendrée.

En vue de cette mise en oeuvre, la présente invention a également pour objet un procédé de calibration tel que décrit dans la revendication 14.

La présente invention permet ainsi la mise en oeuvre d'un système pour le traitement thermique interne de corps certains, par application d'énergie microonde, ce système, grâce à une grande dynamique de niveau de puissance rayonnée au voisinage de la zone à traiter, présentant une grande souplesse d'utilisation pour des applications très variées.

Le système pour le traitement thermique interne de corps certains par application d'énergie micro-onde, objet de la présente invention, est remarquable en ce qu'il comprend un générateur d'énergie micro-onde à une fréquence déterminée et au moins une voie de transmission de l'énergie micro-onde émise par le générateur, chaque voie permettant d'engendrer un signal de traitement micro-onde modulé en amplitude selon une loi de modulation périodique de fréquence f1, l'onde porteuse de l'énergie micro-onde de deux voies consécutives présentant un déphasage de valeur déterminée. Un applicateur micro-onde au moins est associé à au moins une voie et permet d'assurer l'application de l'énergie micro-onde délivrée par cette voie en des points distincts au voisinage de la zone à traiter et un radiomètre, à deux références de température est interconnecté sélectivement à cet applicateur micro-onde, jouant le rôle de capteur et permettant de mesurer la température absolue du point distinct correspondant au voisinage de la zone à traiter. Une console de calcul et d'affichage des puissances micro-onde rayonnées et des températures instantanées des points distincts de la zone à traiter est prévue.

Le système pour le traitement thermique interne de corps certains objet de l'invention trouve application notamment au traitement de tissus vivants, dans les cas non limitatifs d'adénome de la prostate, de ménorragie intra-utérine ou analogue.

Un descripion plus détaillée d'un système pour le traitement thermique interne d'un corps certain et de l'utilisation de celui-ci sera donnée en liaison avec la description ci-après et les dessins dans lesquels :

- la figure 1 représente un schéma synoptique d'un système pour le traitement thermique interne d'un corps certain selon l'invention ;
- la figure 2 représente un schéma synoptique d'un radiomètre à deux références de température ;
- la figure 3a représente un mode de réalisation avantageux non limitatif d'un radiomètre à deux références de température susceptible d'être utilisé pour la mise en oeuvre d'un système pour le traitement thermique interne d'un corps certain selon l'invention tel que présenté en figure 1;
- les figures 3b, 3c et 3d sont des vues partielles relatives à l'utilisation du système pour le traitement thermique interne de corps certains, objet de l'invention, et en particulier à un processus de calibration de ce système, dans des conditions de meilleure précision d'étalonnage antérieurement et/ou au cours d'une séance de traitement ;
- la figure 4 représente un mode de réalisation avantageux non limitatif d'un système pour le traitement thermique interne d'un corps certain plus particulièrement adapté au traitement de l'adénome de la prostate ;
- les figures 5a, 5b et 5c sont relatives à différents modes de réalisation d'applicateurs d'énergie micro-onde, également susceptibles de jouer le rôle de capteur de température, conformément à une caractéristique essentielle de l'objet de l'invention ;
- la figure 6a est relative à l'utilisation du système pour le traitement thermique interne de corps certains selon l'invention, dans le cas du traitement de tissus vivants, en vue de la réduction d'un adénome de la prostate.
- les figures 6b et 6c sont relatives à l'utilisation du système pour le traitement thermique interne de corps certains selon l'invention dans un cas général, et notamment dans le cas de la figure 6a.

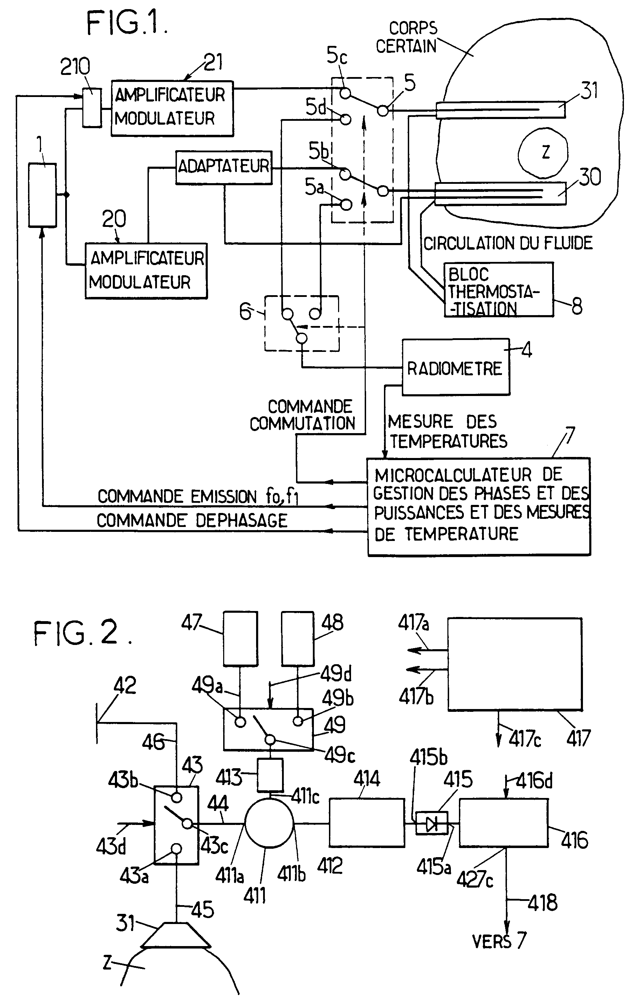

Une description plus détaillée d'un système pour le traitement thermique interne de corps certains par application d'énergie micro-onde sur une zone à traiter, conforme à l'objet de la présente invention, sera donnée en liaison avec la figure 1.

D'une manière générale, ainsi que représenté sur la figure précitée, le système objet de la présente invention comporte un générateur 1 d'énergie micro-onde à une fréquence f0 déterminée. Le générateur 1 peut consister en un oscillateur délivrant une onde progressive à la fréquence de 915 Mz, par exemple.

Le système comporte également, ainsi qu'on l'a représenté sur la figure 1 précitée, au moins une voie, notée 20, 21, de transmission de l'énergie micro-onde émise par le générateur 1. Chaque voie permet d'engendrer un signal de traitement micro-onde modulé en amplitude selon une loi de modulation périodique déterminée de fréquence f1. Sur la figure 1, on a représenté de façon non limitative les voies 20 et 21 constituées par un amplificateur auquel est associé un modulateur d'amplitude, par exemple.

Selon une caractéristique particulièrement avantageuse du système objet de la présente invention, l'onde porteuse de l'énergie micro-onde de 2 voies consécutives, les voies 20 et 21 sur la figure 1, présente un déphasage de valeur déterminée.

Ainsi qu'il sera décrit de manière plus détaillée dans la description, le déphasage introduit au niveau de l'onde porteuse transmise par deux voies consécutives permet, en fait, d'obtenir une très grande dynamique de l'énergie micro-onde appliquée sur la zone Z à traiter.

Ainsi qu'on l'a en outre représenté en figure 1, le système objet de la présente invention comporte au moins un applicateur micro-onde 30, 31, associé à au moins 1 voie. L'applicateur 30 est, par exemple, associé à la voie 20, alors que l'applicateur 31 est associé à la voie 21. Chaque applicateur permet ainsi d'assurer l'application de l'énergie micro-onde délivrée par la voie correspondante en des points distincts au voisinage de la zone Z à traiter.

Selon une caractéristique particulièrement avantageuse du système, objet de la présente invention, celui-ci comporte un radiomètre 4 à deux références de température. Le radiomètre 4 peut être connecté sélectivement à l'un ou l'autre des capteurs micro-onde 30, 31, chaque applicateur jouant alors le rôle de capteur de température et permet ainsi de mesurer la température absolue du point distinct correspondant au voisinage de la zone Z à traiter.

Sur la figure 1, on a représenté l'interconnexion sélective du radiomètre 4 à deux références de température à l'un et/ou à l'autre des applicateurs 30, 31, par l'intermédiaire d'un premier commutateur micro-onde multivoies 5 et d'un deuxième commutateur micro-onde multivoies 6. L'utilisation de deux commutateurs micro-onde multivoies 5 et 6, telle que représentée en figure 1, ne préjuge en aucun cas de l'utilisation d'un seul commutateur micro-onde multivoies pour assurer une fonction analogue.

Ainsi qu'on l'a en outre représenté en figure 1, le système objet de la présente invention comprend une console de calcul et d'affichage des puissances micro-onde rayonnées et des températures instantanées des points distincts de la zone Z à traiter. On notera bien sûr que la console de calcul et d'affichage 7 comprend, par exemple, un micro-ordinateur muni de ses ressources périphériques, ce micro-ordinateur étant interconnecté, d'une part, au générateur 1 d'énergie micro-onde afin de commander l'émission de l'onde porteuse du signal micro-onde chargée de délivrer l'énergie thermique de traitement, à un ou plusieurs circuits de déphasage tel que le circuit 210 représenté sur la voie de transmission 21, le déphasage étant commandé par l'intermédiaire du micro-ordinateur de la console de calcul et d'affichage 7 en fonction des applications considérées, ainsi qu'il sera décrit ultérieurement dans la description. En outre, ainsi qu'on le comprendra aisément, la console de calcul et d'affichage 7 permet de commander, par l'intermédiaire d'une interconnexion, les commutateurs micro-onde 5 et 6 de façon à assurer la commutation des applicateurs 30 et 31 par exemple, soit en liaison avec le radiomètre 4 à deux références de température, soit en liaison avec les voies respectives 20 ou 21.

On notera ainsi que le fonctionnement du système objet de la présente invention est particulièrement simplifié par rapport au dispositif de l'art antérieur dans la mesure où le ou les applicateurs 30 ou 31 formant également capteurs de température, leur implantation en fonction de l'application considérée n'est pas obérée par l'implantation nécessaire de capteurs de température distincts, ce qui permet bien entendu d'améliorer notoirement la précision du traitement ainsi conduit, tant en ce qui concerne la valeur de la température effectivement produite dans la zone Z à traiter que de la mesure de cette température au point d'application effectif de l'énergie micro-onde engendrant l'hyperthermie.

Une des composantes permettant d'obtenir la précision la plus grande relativement à la température atteinte par la zone Z à traiter consiste, conformément à l'un des objets de la présente invention, en l'utilisation d'un radiomètre 4 à deux références de température.

Une description plus détaillée d'un tel type de radiomètre comme de l'état de la technique qui est décrit dans le document FR-A-2650390, sera donnée en liaison avec la figure 2.

Selon la figure précitée, un tel radiomètre existant à deux références 4 peut comprendre un premier commutateur micro-onde 43 comprenant deux voies d'entrée notées 43a, 43b. Ces voies d'entrée sont connectées, l'une, 43a, à la sortie d'un applicateur par l'intermédiaire d'un commmutateur micro-onde multivoies, noté 43, et l'autre, 43b, à un court-circuit micro-onde, noté 42. Une sortie 43c du commutateur micro-onde 43 peut être connectée à l'une ou l'autre des deux entrées précitées.

Ainsi qu'on l'a représenté en figure 2, le radiomètre 4 à deux références de température comporte en outre un circulateur 411 comprenant 3 voies, 411a, 411b, 411c. La première voie 411a est connectée à la sortie du premier commutateur micro-onde 43 et la deuxième voie 411b est connectée à l'entrée d'un ensemble de traitement électronique micro-onde noté 414, 415, 416, 417. La troisième voie 411c du circulateur 411 constitue une entrée de référence, c'est-à-dire une entrée recevant le signal délivré par l'une des références de température ainsi qu'il sera décrit ultérieurement dans la description.

Sur la figure 2, on pourra observer que le radiomètre 4 à deux références comporte également au moins 2 sources, 47, 48, de référence de température internes et un second commutateur micro-onde, 49. Le second commutateur micro-onde 49 comporte au moins deux voies d'entrée, 49a et 49b, connectées chacune à la sortie d'une des sources de référence, 47, 48 respectivement. Le deuxième commutateur micro-onde 49 comprend une sortie 49c connectée à la troisième entrée de référence du circulateur 411c.

En outre, le radiomètre à deux références de température comprend un circuit de commande de synchronisation, 417, lequel agit tant sur le premier commutateur micro-onde, 43, que sur le second, 49, pour obtenir au moins quatre signaux de sortie successifs différents, chacun des signaux précités correspondant à chacune des possibilités de connexion des deux commutateurs micro-ondes respectifs 43 et 49.

Ainsi, le premier commutateur micro-onde 43 comprend 2 voies d'entrée, alors que le second commutateur micro-onde 49 comporte au moins 2 voies d'entrée.

Ainsi qu'on l'observera sur la figure 2, la sortie, 411b, du circulateur 411 est reliée à un amplificateur micro-onde, 414, et la sortie de l'amplificateur micro-onde 414 est elle-même reliée à un détecteur micro-onde, 415, à une entrée, notée 415b. La sortie, 415a, du détecteur micro-onde 415 est reliée à un circuit d'échantillonnage, 416, lequel délivre des valeurs échantillonnées des signaux délivrés par le détecteur micro-onde 415 par l'intermédiaire d'une sortie, 418, à la console de calcul et d'affichage 7.

On notera d'ailleurs que la console de calcul et d'affichage 7 permet, sous la commande du circuit de commande et de synchronisation, 417, notamment, de calculer à partir des valeurs échantillonnées des 4 signaux successifs précités la température au niveau de l'applicateur considéré, indépendamment du coefficient de réflexion de l'applicateur vis-à-vis de la zone Z à traiter et des pertes des commutateurs micro-onde et des câbles d'interconnexion.

On notera en particulier que la valeur de la température absolue T de la région Z à traiter peut être déterminée, à partir des températures de références T1 et T2 connues des éléments résistifs connectés au deuxième commutateur 49, et représentés par les références 47 et 48 sur la figure 2, et des tensions disponibles en sortie de l'échantillonneur 416, ceci indépendamment du coefficient de réflexion r et de la largeur P de la bande passante du dispositif.

Pour une description plus détaillée du fonctionnement d'un radiomètre à deux références tel qu'incorporé dans le système objet de la présente invention et représenté en figure 2, on pourra se reporter avantageusement à la demande de brevet français n°89 10148 déposée au nom de la demanderesse le 27 juillet 1989 et publiée le 1er février 1991 sous le n° 2 650 390.

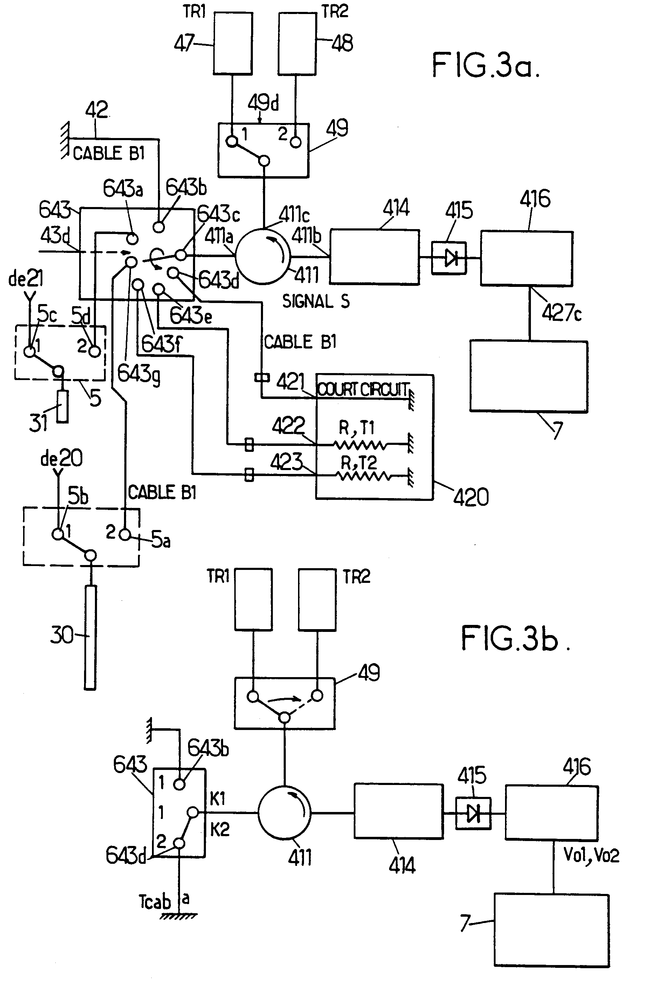

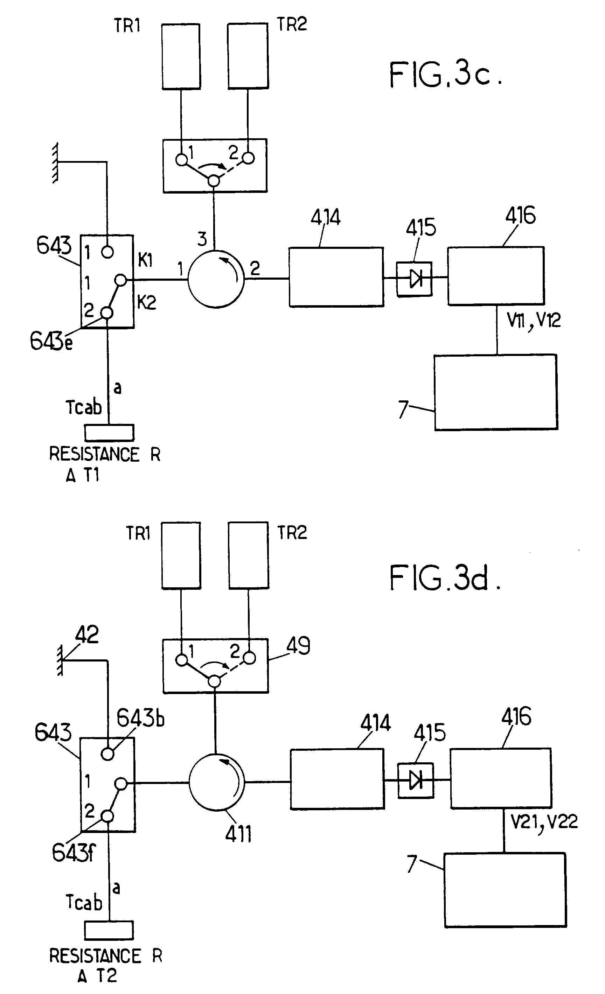

Un mode de réalisation préférentiel d'un système conforme à l'objet de la présente invention incorporant un radiomètre 4 à deux références de température particulièrement avantageux sera maintenant décrit en liaison avec la figure 3a.

D'une manière générale, on notera que le dispositif de mesure de température constitué par le radiomètre 4 à deux référence de température se compose principalement du commutateur micro-onde 43 représenté en figure 2 à deux voies, ce commutateur pouvant être réalisé par un commutateur de type général MICROWAVE modèle F 9120 ou F 9220 adapté à la fréquence de travail reliant l'entrée 411a du circulateur, soit à l'antenne de l'applicateur 30 ou 31, soit au court-circuit 42 précédemment décrit dans la description.

Les câbles de liaison entre le commutateur micro-onde 43 et l'applicateur correspondant, le court-circuit 42 et ce même commutateur micro-onde doivent être à très faible perte et de longueur égale, notée B1 sur la figure 3a, afin de présenter les mêmes coefficients de transmission des signaux correspondants.

Le circulateur 411 est un circulateur à large bande, la bande de fréquence de celui-ci étant au moins égal à 1GHz autour de la fréquence centrale du système, laquelle peut être prise égale, pour le radiomètre à deux références 4, à 1,2 ou 3 ou 9 GHz. Le circulateur précité peut être, par exemple, un circulateur à 3 voies de type NARDA COS 2652 ou analogue.

On notera enfin que le commutateur micro-onde 49 permet de commuter les 2 sources de bruit à température de référence 47, 48, lesquelles fournissent les températures de référence TRj, avec j prenant les valeurs 1 ou 2.

Une combinaison des positions de commutation des commutateurs micro-onde 43 et 49, telles que décrites par la table de vérité donnée ci-après, permet d'obtenir les différents signaux S1, S2 et S3, S4 en sortie du bloc d'échantillonnage 416.

```
          P o s i t i o n    d e    c o m m u t a t i o n

              _____

                49              49a                     49b
           43
   P   c    _____
   o   o
   s   m
   i   m   43a      S2 = K.P.[ r.TR1        S4 = K.P.[ r.TR2
   t   u                + (1-r)T ]              + (1-r)T ]
   i   t
   o   a    _____
   n   t
   d   o   43b      S1 = K.P.TR1            S3 = K.P.TR2
   e   n    _____
```

Dans les relations donnant les valeurs des signaux précités, on notera que K désigne la constante de Boltzman, P désigne la valeur de la bande passante du radiomètre à deux références 4 et r désigne la valeur du coefficient de réflexion de l'applicateur 30 ou 31 considéré. Les signaux échantillonnés précités sont alors mémorisés au niveau du micro-ordinateur constituant la console de calcul et de visualisation 7 et une combinaison linéaire de ces signaux obtenue à partir des expressions ci-après :

$$Va \simeq S1 - S2$$

$$Vb \simeq S3 - S4$$

permet d'obtenir la valeur de la température absolue T de la zone Z à traiter par la relation :

$$T = \frac{V_a TR2 - V_b TR1}{V_a - V_b}.$$

On notera ainsi que la valeur de la température absolue T de la région Z à traiter est donc calculable à partir des températures de référence TR1 et TR2, et ceci en toute indépendance relativement au coefficient de réflexion r et à la largeur P de la bande passante du radiomètre à deux références.

Pour une description plus détaillée du radiomètre à deux référence 4 mis en oeuvre dans le système objet de la présente invention, on pourra utilement se reporter à la description de la demande de brevet français déjà mentionnée précédemment.

Ainsi qu'on l'a représenté en outre en figure 3a, le radiomètre 4 à deux références comprend un module d'étalonnage, 420, à trois sorties. Une première sortie, 421, du module d'étalonnage 420 est reliée à un court-circuit électrique dans la bande de fréquence micro-onde du radiomètre 4, une deuxième sortie, 422, du module d'étalonnage est reliée à une première résistance calibrée de valeur déterminée dans la bande de fréquence du radiomètre, cette résistance étant portée premièrement à une température T1 de valeur déterminée. Enfin, la troisième sortie 423 du module d'étalonnage 420 est reliée à une deuxième résistance calibrée de même valeur déterminée dans la bande de fréquence du radiomètre 4, cette deuxième résistance étant en fonctionnement portée à une température T2 déterminée.

On notera que de manière avantageuse, le commutateur micro-onde 43 du radiomètre à deux références tel que représenté en figure 2 peut alors être remplacé par un commutateur micro-onde multivoies noté 643, la référence 643 désignant en fait une combinaison du commutateur micro-onde à deux voies 43 de la figure 2 et du commutateur micro-onde multivoies 6 de la figure 1 par exemple.

On notera bien sûr que le commutateur micro-onde multivoies 643 permet de relier sélectivement à l'entrée formant entrée de mesure du radiomètre 4 à deux références, soit l'un des applicateurs micro-onde 30 ou 31, soit l'une des sorties du module d'étalonnage. Ainsi, le commutateur micro-onde multivoies 643 est un commutateur à 6 entrées et une sortie, les 6 entrées étant notées 643a, b, d, e, f, g et la sortie étant notée 643c par analogie avec le commutateur micro-onde 643 de la figure 2. Les entrées 643d, e, f, sont reliées respectivement à la première 421, deuxième 422, et troisième 423 sortie du module d'étalonnage 420 à trois sorties. En outre, les entrées 643g, a et b, sont respectivement reliées aux bornes 5a, 5d, du commutateur multivoies 5 tel que représenté en figure 1 et au court-circuit 42.

On notera ainsi que dans le cadre de la figure 3a, l'entrée de mesure du radiomètre 4 à deux références est réalisée soit par la borne 643g, soit par la borne 643a du commutateur multivoies 643.

On notera de manière avantageuse que les câbles d'interconnexion entre le commutateur multivoies 643, les entrées de celui-ci précédemment décrites et leurs différents points de liaison peuvent avantageusement être constitués par des câbles d'égale longueur notés B1 ainsi que représenté sur la figure 3a. La longueur des câbles est ainsi choisie de façon à réaliser un équilibrage du dispositif en ce qui concerne les pertes introduites par les câbles de connexion précités.

Une description du mode opératoire permettant la mise en oeuvre d'un calibrage automatique d'un système pour le traitement thermique interne de corps certains, objet de la présente invention, selon le mode de réalisation de la figure 3a sera maintenant donnée en liaison avec les figures 3b à 3d représentant des vues partielles du radiomètre de la figure 3a en fonction de l'étape de calibration concernée.

Dans le processus de calibration mis en oeuvre, on notera que la calibration peut être effectuée à partir de l'un des applicateurs 30, 31 de manière semblable. Dans tous les cas, l'entrée de mesure du radiomètre à deux références 4 sera alors considérée comme la borne 643c du commutateur micro-onde multivoies 643, laquelle peut être interconnectée à l'une des bornes d'entrée précédemment décrite dans la description du commutateur multivoies 643.

Le processus de calibration, conformément à un objet de la présente invention, consiste alors avantageusement, ainsi qu'illustré en liaison avec la figure 3b, en premier lieu, à connecter l'entrée de mesure du radiomètre à deux références à la première sortie 421 du module d'étalonnage 420, l'entrée de mesure du radiomètre 4 à deux références étant ainsi placée en court-circuit. Le deuxième commutateur multivoies 49 du radiomètre à deux références 4 est alors connecté sur la première, TR1, puis sur la deuxième, TR2, référence de température ainsi qu'illustré en figure 3b. Les valeurs correspondantes Vo1 et Vo2 du signal délivré par le circuit d'échantillonnage 416 sont alors mesurées et mémorisées.

Le processus de calibration consiste en deuxième lieu à connecter l'entrée de mesure du radiomètre 4 à deux références à la deuxième sortie 422 du module d'étalonnage 420. L'entrée de mesure précitée est alors connectée à la résistance de valeur déterminée portée à la température T1 de valeur déterminée. Le deuxième commutateur multivoies 49 est alors connecté sur la première TR1, puis sur la deuxième TR2, référence de température. Les valeurs V11 et V12 correspondantes du signal délivré par le circuit d'échantillonnage 416 sont alors mesurées et mémorisées.

En troisième lieu, le processus de calibration consiste à connecter l'entrée de mesure du radiomètre à deux références à la troisième sortie 423 du module d'étalonnage 420. L'entrée de mesure précitée est alors connectée à la résistance de valeur déterminée portée à la température T2 de valeur déterminée. Le deuxième commutateur multivoies 49 du radiomètre 4 à deux références est alors connecté sur la première TR1, puis sur la deuxième TR2, référence de température. Les valeurs V21 et respectivement V22 correspondantes du signal délivré par le circuit d'échantillonage 416 sont alors mesurées et mémorisées.

On notera que les valeurs mémorisées précitées vérifient chacune une relation de la forme :

$$Vij = K.\Delta F(a.K2 \times T \times (1-\rho) + Tcab.K2(1-a).(1-\rho) - a^2\rho + TRj(a^2+K^22.\rho-K^21).$$

On notera que dans cette relation :

Vij        désigne chaque valeur mémorisée précitée,

K        désigne le produit du gain G de la chaîne par la constante de Boltzman KB,

ΔF        désigne la bande passante du radiomètre à deux références,

TRj        désigne soit la première TR1, soit la deuxième TR2, températures de référence, pour j prenant les valeurs 1, respectivement 2,

K1 et K2        désignent les coefficients de perte du premier commutateur micro-onde 643 pour les voies 1 et 2,

a        désigne les pertes du câble permettant d'assurer la liaison du câble entre entrée de mesure du radiomètre à deux références 4 et les différentes entrées du module de calibration 420,

Tcab        désigne la température du câble de liaison avec ρ = 0 lors de l'interconnexion de l'entrée du radiomètre à deux références 4 sur la deuxième ou troisième entrée, 422, 423 et sur la résistance correspondante déterminée du module d'étalonnage 420, et ρ = 1 lors de l'interconnexion de l'entrée du radiomètre 4 à deux références sur la première entrée 421 et sur le court-circuit correspondant du module d'étalonnage 420.

Les équations linéaires ayant les valeurs mémorisées précitées Vij et les paramètres du dispositif précédemment mentionné peuvent alors être résolus de manière numérique au moyen du micro-calculateur constituant la console de calcul et d'affichage 7 pour déterminer les paramètres précités.

On notera d'une manière générale que les différentes étapes du processus de calibration précédemment décrites peuvent être permutées sans sortie du cadre de la présente invention en raison du fait notamment que les relations vérifiées par les valeurs de tension échantillonnées Vij précédemment décrites sont linéaires, les paramètres physiques du dispositif étant considérés comme des variables indépendantes.

On notera d'ailleurs que le processus de calibration précédemment décrit peut être mis en oeuvre plus particulièrement dans le cadre d'un étalonnage réalisé en laboratoire de la manière ci-après lorsque les deux températures T1 et T2 auxquelles sont portées les résistances du module de calibration 420 sont ajustables. Dans un tel cas le processus de calibration peut consister, de manière avantageuse :

- en premier lieu, à effectuer l'étape décrite en premier lieu précédemment dans la description dans le cadre du processus de calibration.
- En deuxième lieu sont effectuées les étapes précédemment décrites en deuxième et troisième lieu dans le processus de calibration antérieur relativement aux températures de références TR1, successivement TR2, la température ajustable T1, T2, c'est-à-dire l'une des deux, étant ajustée progressivement de façon à obtenir une valeur nulle pour les valeurs correspondantes V11, V12, ou V21, V22, délivrées par le circuit d'échantillonnage 416, pour des valeurs correspondantes dites températures de zéro, et notées respectivement TZ1, TZ2, des températures ajustables T1 ou T2. Chaque température de zéro vérifie une relation du type :

$$TZj = (TRj.K1^2 - Tcab.K2(1-a) + Voj) / (a.K2.K.\Delta F).$$

On notera que dans cette relation, j désigne bien entendu l'indice pouvant prendre la valeur 1 ou 2 relative à la référence de température effectivement utilisée.

De même, K désigne le produit du gain de la chaîne par la constante de Boltzman KB.

On notera que dans le mode de réalisation de la figure 3a, l'existence du court-circuit 42, lequel est relié à la borne 643b du commutateur multivoies 643, et du court-circuit relié à la sortie 421 du module de calibration 420 implique l'utilisation de câbles de connexion identiques, afin de réaliser un bon équilibrage physique de l'ensemble du système. Cet équilibrage peut être vérifié par la comparaison des tensions ou signaux échantillonnés délivrés par le module d'échantillonnage 416, une valeur identique ou sensiblement identique, aux erreurs de mesure près, de ces signaux délivrés respectivement par le module de détection échantillonnée 416 lors de la commutation sur l'un ou l'autre des court-circuits indiquant un équilibrage satisfaisant de l'ensemble du dispositif.

Bien entendu, ainsi que représenté en figure 3d, l'un des court-circuits peut être supprimé, et dans ce cas, le commutateur micro-onde multivoies 643 comporte une borne d'entrée en moins, le court-circuit du module de calibration 420 et la première sortie 421 de celui-ci étant par exemple supprimés. Le module de calibration 420 est alors un module de calibration à deux sorties, 422 et 423. Une telle modification du module de calibration permet de réaliser en fait un module de calibration équivalent à celui précédemment décrit dans la description.

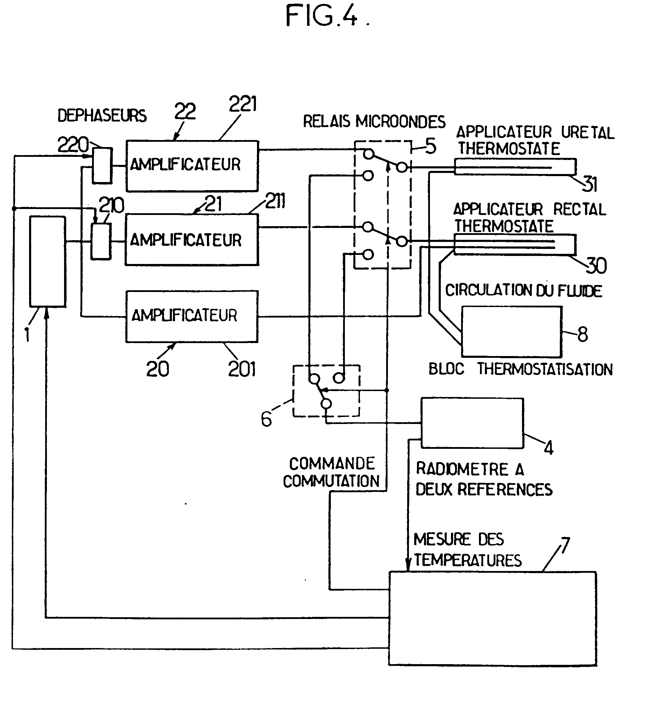

Une description plus détaillée d'un système pour le traitement thermique interne de corps certains conforme à l'objet de la présente invention, plus particulièrement adapté au traitement de tissus vivants, en particulier au traitement d'un adénome de la prostate, sera maintenant donnée en liaison avec la figure 4.

Ainsi qu'on l'a représenté sur la figure précitée, le système objet de la présente invention comprend, de manière avantageuse, une première, 20, une deuxième, 21, et une troisième, 22, voies de transmission connectées en parallèle en sortie du générateur 1 d'énergie micro-onde. Un premier, 30, et un deuxième, 31, applicateur micro-onde sont prévus, le premier applicateur micro-onde, 30, applicateur rectal, étant relié, d'une part à la sortie de la première voie, 20, et d'autre part, sélectivement à la sortie de la deuxième voie, 21, ou à l'entrée de mesure du radiomètre 4 à deux références par l'intermédiaire d'un premier et d'un deuxième commutateurs micro-onde multivoies, lesquels sont notés 5 et 6 sur la figure 4.

Le deuxième applicateur, 31, applicateur urétral, est également sélectivement relié à la sortie de la troisième voie, 22, ou d'autre part, à l'entrée de mesure du radiomètre à deux références par l'intermédiaire du premier commutateur multivoies 5 et du deuxième commutateur multivoie 6.

On notera en particulier que les premier et deuxième applicateurs 30, 31, sont thermostatés par l'intermédaire d'un bloc de thermostatisation 8, lequel délivre un fluide de thermostatisation à chacun des applicateurs précités. Le bloc de thermostatisation ne sera pas décrit en détails, car il correspond à un élément constitutif connu de l'homme de métier.

On notera cependant que les deuxième et troisième voies, 20, 21, comportent chacune un circuit déphaseur programmable, 210, 211, permettant de déphaser l'onde porteuse de l'énergie micro-onde transmise vers les applicateurs, 30, 31, correspondants.

On notera en outre que chaque voie, 20, 21, 22, comporte un amplificateur correspondant, 201, 211, 221, de l'énergie micro-onde, cet amplificateur pouvant jouer le rôle de modulateur de façon à moduler l'onde porteuse selon une suite d'impulsions de rapport cyclique déterminé, le rapport cyclique permettant ainsi de déterminer l'énergie micro-onde effectivement appliquée au niveau de chaque applicateur. Les dispositifs amplificateurs modulateurs micro-onde précédemment mentionnés ne seront pas décrits en détail, car il peuvent être réalisés par tout dispositif amplificateur modulateur normalement disponibles dans le commerce pour assurer l'amplification et la modulation d'énergie micro-onde dont la fréquence f0 est égale sensiblement à 915 Mz.

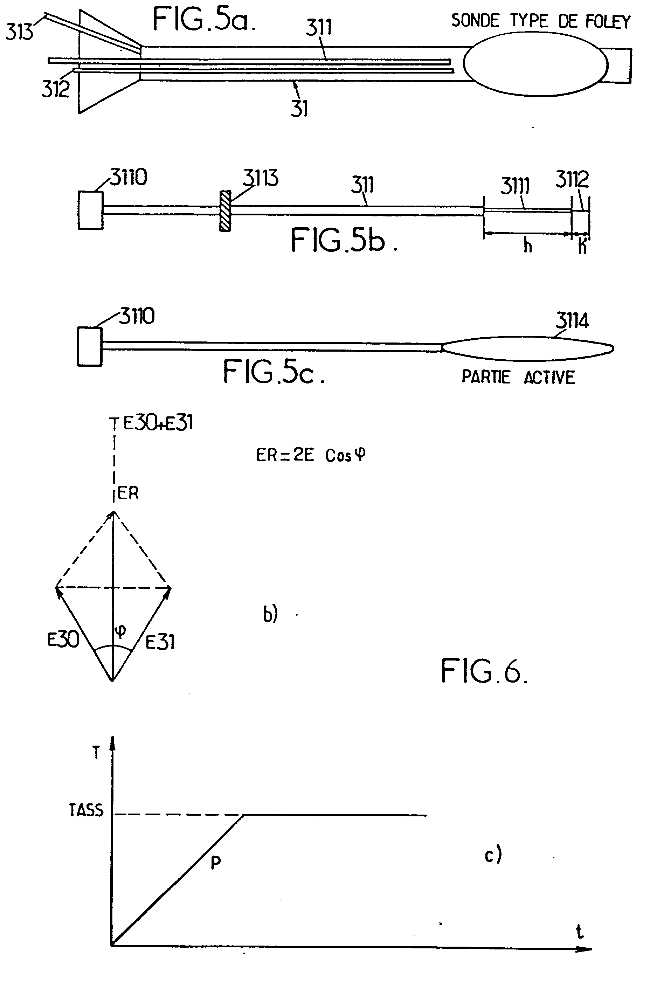

Une description plus détaillée de l'applicateur urétral 31 sera donnée en liaison avec les figures 5a à 5c.

Selon les figures précitées, l'applicateur urétral comprend, dans une sonde de type sonde de Foley, une antenne filaire 311 connectée au commutateur correspondant par un connecteur micro-onde, 3110a, et un câble coaxial, 3110b. L'applicateur urétral comporte également un tuyau d'entrée d'eau, 312, et un tuyau de sortie d'eau, 313, dans la sonde, l'eau étant délivrée par le bloc de thermostatisation 8, et permettant ainsi d'assurer une circulation d'eau jouant le rôle de fluide de thermostatisation.

Ainsi qu'on le notera à l'observation de la figure 5b, l'antenne filaire constituée par le câble micro-onde 311 comporte une butée, 3113, disposée à une distance convenable de l'extrémité de l'antenne filaire précitée, cette butée étant destinée à régler la longueur effective de l'antenne filaire dans la sonde de type de Foley. La butée 3113 peut être réalisée par un matériau plastique, par exemple, collé sur le câble micro-onde 3110b.

Ainsi qu'on l'a également représenté en figure 5b, on notera que l'antenne filaire 311 est formée par le câble coaxial présentant successivement à son extrémité dans le sens de propagation directe de l'énergie micro-onde, une partie, 3111, de longueur h sur laquelle le blindage du câble coaxial a été supprimé, l'âme centrale du câble coaxial étant sur cette partie recouverte par le matériau diélectrique du câble. Une partie, 3112, de longueur h' est formée par l'âme centrale du câble coaxial totalement dénudé. Les longueurs h et h' sont déterminées en fonction de la fréquence f0 de l'onde porteuse de l'énergie micro-onde et de la dimension de la zone à chauffer.

On notera que, en vue de réaliser les applications en gynécologie, l'applicateur décrit en liaison avec les figures 5a et 5b peut être avantageusement modifié ainsi que représenté en figure 5c.

Dans ce cas, les parties de longueur h et h' sont recouvertes d'une coiffe, 3114, en matériau diélectrique. La coiffe présente une forme oblongue afin d'assurer une adaptation d'impédance de l'antenne filaire au tissu environnant.

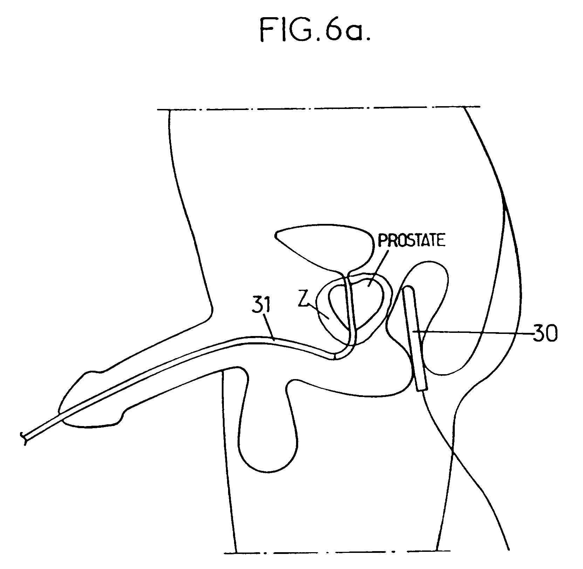

Une description détaillée d'un mode opératoire, soit d'un mode d'utilisation du système objet de la présente invention tel que représenté en figure 4, plus particulièrement dans le cas d'un traitement d'un adénome de la prostate, sera donné en liaison avec les figures 6a à c.

Dans le cas précité, le processus de traitement proprement dit comprend, après implantation des applicateurs rectal et urétral 30, 31, ainsi que représenté en figure 6a dans la ou les cavités physiologiques adéquates correspondantes, notamment des étapes consistant en une émission, vers le ou les applicateurs 30, 31, précités d'un niveau d'énergie micro-onde déterminé et de modulation de la phase relative de l'onde porteuse de l'énergie micro-onde transmise par deux voies consécutives selon un programme de modulation déterminé pour moduler le niveau d'énergie transférée au niveau de la zone à traiter Z.

Bien entendu, de l'observation de la figure 6a, on comprendra que la zone Z à traiter englobe la prostate soumise au traitement, cette zone Z étant située entre les deux applicateurs après implantation de ces derniers.

Ainsi qu'on l'a représenté en figure 6b, la modulation de phase, c'est-à-dire le choix d'une valeur $\varphi$ de la phase relative du champ électrique rayonné par le premier et le deuxième applicateurS, par exemple, 30, 31, permet d'engendrer au niveau de la zone Z à traiter un champ électrique résultant, ER, dont l'amplitude vérifie la relation $ER = 2E.\cos\varphi$, ce qui a pour effet de permettre l'application d'une énergie micro-onde proportionnelle au carré de l'amplitude précitée, soit proportionnelle à quatre fois l'amplitude du champ électrique rayonnée par l'un des applicateurs. On obtient ainsi une très grande dynamique de l'énergie effectivement appliquée au niveau de la zone Z à traiter, cette dynamique étant dans un rapport 2 par rapport à la dynamique d'énergie appliquée en l'absence de modulation. La modulation programmée de la phase relative de l'onde porteuse de l'énergie micro-onde et donc du champ électrique rayonné par chaque applicateur peut être effectuée continuement ou par incréments discrets, ceci afin de moduler la quantité d'énergie appliquée à la zone Z à traiter et finalement l'accroissement de température apporté à la zone précitée.

Sur la figure 6c, on a représenté à titre d'exemple non limitatif une loi d'évolution de la montée en température de la zone Z à traiter vers une température constante notée Tass, laquelle peut être maintenue par l'émission périodique de l'un et/ou de l'autre applicateur 30, 31.

On notera bien entendu que, d'une part, la température d'asservissement Tass ainsi que la pente p, ou la loi de convergence vers cette température d'asservissement, sont déterminées par le praticien en fonction de l'application considérée.

Selon une caractéristique avantageuse, la pente p ou la loi de convergence vers la température d'asservissement peut être choisie de façon à correspondre à une fonction monotone croissante de la température en fonction du temps par incrément de température $\Delta T$ compris entre 0,8°C à 1,2°C par minute.

De préférence, et selon un aspect particulièrement avantageux du système objet de la présente invention, la puissance rayonnée, laquelle correspond à une puissance électrique rayonnée maximale rayonnée de 50 watts peut alors être ajustée de façon à vérifier pour les incréments de puissance apportés par chaque rayonnement successif la relation :

$$\Delta P = M.\Delta T + N.\Delta T \text{ théorique}/\Delta T \text{ atteinte.}$$

On notera bien sûr que dans la relation précitée,

$\Delta P$ représente l'incrément de puissance délivrée par l'un et/ou l'autre des applicateurs 31 ou 30,

M représente un coefficient de proportionnalité dépendant de la zone, et finalement de l'organe à traiter, et des applicateurs utilisés,

$\Delta T$ représente l'accroissement de température par rapport à la mesure antérieure au moyen du radiomètre à deux références,

N représente un coefficient de proportionnalité dépendant également de l'organe et des applicateurs utilisés ainsi que de la loi de croissance de température choisie par le praticien,

$\Delta T$ théorique
est une valeur mémorisée au niveau du micro-ordinateur constituant la console de calcul et d'affichage 7, ces valeurs mémorisées correspondant à l'application considérée et constituant, pour cette application, une base de données à la disposition du praticien, et, $\Delta T$ atteinte
étant l'accroissement de température mesuré par le radiomètre à deux références entre deux mesures de température séparées par l'application d'une quantité d'énergie micro-onde donnée.

On notera bien entendu que, à la base de données précitée, sont incorporées les valeurs de M et N ainsi que des valeurs représentatives de la loi de croissance de température, l'ensemble étant à la disposition du praticien.

On notera en particulier que pour des valeurs de déphasage données, le point le plus chaud de la zone Z traitée se déplace soit en avant de l'urètre ou en arrière du rectum en tenant compte de l'épaisseur de la prostate. Donc, pour une prostate de volume déterminé par échographie, il est possible de calculer alors les différentes phases et le rapport des puissances micro-onde rayonnées par chacune des voies pour obtenir une efficacité thermique maximale. A titre d'exemple non limitatif, lorsque les trois voies délivrent une puissance micro-onde en phase, les puissances délivrées par chacune des voies étant identiques, la zone la plus chaude de situe sensiblement à mi-distance entre l'urètre et la paroi rectale.

Selon un autre mode de traitement, celui-ci peut consister à faire varier en fonction du temps la phase de l'onde porteuse délivrée par chacune des voies et en particulier de deux voies consécutives, soit de façon continue par phase variable entre 0 et 180 °, soit avec des valeurs fixées de déphasage, par exemple.

Ainsi, un mode de traitement peut consister pour :

t = 0                 applicateur urétral 31        phase = 0°
                      applicateur rectal voie 21     phase = 0°
                      applicateur rectal voie 20     phase = 120°
t = 4 secondes        permutation cyclique          phase urétrale = 120°,
                      les autres phases étant prises égales à 0°.

On notera que le choix du temps de séquencement est fonction de la géométrie et du volume de l'organe & traiter et donc de la zone Z soumise à l'hyperthermie. Ce séquencement, pour produire un effet thermique optimum, selon une étude théorique menée, doit être compris entre 3 et 9 secondes.

On notera que les commutateurs micro-onde multivoies permettent de commuter les différents applicateurs, ceux-ci fonctionnant soit comme applicateurs d'énergie micro-onde, soit comme détecteurs ou capteurs de température. Par exemple, dans certains cas, il est préférable de chauffer les tissus avec l'un des applicateurs, par exemple l'applicateur rectal, et de mesurer la température par radiométrie au moyen de l'applicateur urétral et/ou une des voies de l'applicateur rectal ou réciproquement.

On a ainsi décrit un système pour le traitement thermique interne de corps certains par application d'énergie micro-onde particulièrement performant, ce système, en raison de l'utilisation d'un radiomètre à deux références de température, permettant de déterminer avec grande précision la température d'une zone à traiter. En outre, le système objet de la présente invention présente une très grande souplesse d'utilisation, en raison du fait que les applicateurs micro-onde sont également utilisés comme capteurs de température au point de rayonnement de l'énergie micro-onde précitée. La très grande souplesse d'utilisation du système objet de la présente invention est également obtenue grâce à la mise en oeuvre d'une modulation de phase de l'énergie micro-onde délivrée par deux applicateurs consécutifs, cette modulation de phase permettant notamment au praticien de provoquer un déplacement du point le plus chaud de la zone traitée, et donc d'envisager ainsi des traitements spécifiques de la zone précitée.

## Revendications

1.  Système pour le traitement thermique interne de corps certains par application d'énergie micro-onde sur une zone à traiter, ce système comprenant

    -   un générateur (1) d'énergie micro-onde à une première fréquence déterminée, au moins deux voies de transmission (20, 21; 20, 21, 22) de l'énergie micro-onde émise par le générateur (1), chaque voie permettant d'engendrer un signal de traitement micro-onde modulé en amplitude selon une loi de modulation périodique déterminée à une deuxième fréquence déterminée, l'onde porteuse de l'énergie micro-onde de deux voies consécutives présentant un déphasage de valeur déterminée
    -   au moins un applicateur (30, 31) micro-onde associé à chacune desdites au moins deux voies permettant d'assurer l'application de l'énergie micro-onde délivrée par lesdites voies en des points distincts au voisinage de la zone (Z) à traiter,
    -   des moyens (7) de calcul et d'affichage des puissances micro-onde rayonnées et des températures instantanées des points distincts de la zone à traiter,
    -   un radiomètre (4) à deux références internes de température interconnecté sélectivement audit au moins un applicateur micro-onde par voie jouant le rôle de capteur et permettant de mesurer la température absolue du point distinct correspondant au voisinage de la zone à traiter par l'application de l'énergie micro-onde, caractérisé en ce qu'il comprend également,
    -   un module d'étalonnage (420) à trois sorties relié audit radiomètre (4) à deux références internes;
    -   une première sortie (421) du module d'étalonnage étant reliée à un court-circuit électrique dans la bande de fréquence micro-onde du radiomètre,
    -   une deuxième sortie (422) du module d'étalonnage étant reliée à une première résistance calibrée de valeur déterminée dans la bande de fréquence du radiomètre, cette résistance étant portée en fonctionnement à une première température T1 de valeur déterminée,
    -   une troisième sortie (423) du module d'étalonnage étant reliée à une deuxième résistance calibrée de même valeur déterminée dans la bande de fréquence du radiomètre, cette deuxième résistance étant, en fonctionnement, portée à une température T2, déterminée,
    -   un commutateur micro-onde multivoies (643) permettant de relier sélectivement à l'entrée du commutateur micro-onde formant entrée de mesure dudit radiomètre à deux références soit l'un des applicateurs micro-onde (30, 31) associé auxdites voies, soit l'une des sorties dudit module d'étalonnage (420).

2.  Système selon la revendication 1 caractérisé en ce que ledit radiomètre à deux références comprend :

- un premier commutateur micro-onde (43) comprenant deux voies d'entrée connectées, l'une (43a) à la sortie d'un applicateur (30, 31) par l'intermédiaire d'un commutateur multivoies, et l'autre (43b) au court-circuit micro-onde et une sortie (43c) connectable à l'une ou l'autre des deux entrées, un circulateur (411) avec trois voies successives, la première (411a) étant connectée à la sortie du premier commutateur micro-onde et la deuxième (411b) à l'entrée d'un ensemble de traitement électronique micro-ondes (414, 415, 416, 417), la troisième voie (411c) constituant une entrée de référence,
- au moins deux sources (47, 48) de référence de température internes et un second commutateur micro-onde (49) avec au moins deux voies d'entrée connectées chacune à la sortie desdites au moins deux sources de référence (47, 48) et une sortie connectée à ladite troisième entrée de référence du circulateur (411), ledit ensemble de traitement électronique comportant :
- un circuit de commande de synchronisation (417) agissant tant sur le premier commutateur micro-onde que sur le second commutateur micro-onde pour obtenir au moins quatre signaux de sortie successifs différents, chacun desdits au moins quatre signaux correspondant à chacune des au moins quatre possibilités de connexion des deux commutateurs micro-onde, le premier à deux voies d'entrée et le second à au moins deux voies d'entrée,
- un détecteur micro-onde (415) à une entrée connectée par l'intermédiaire d'un amplificateur micro-onde (414) à la sortie du circulateur,
- un circuit d'échantillonnage (416) connecté en sortie du détecteur micro-onde et délivrant des valeurs échantillonnées des signaux délivrés par le détecteur micro-onde auxdits moyens de calcul et d'affichage, lesdits moyens de calcul, sous la commande du circuit de commande et de synchronisation permettant, à partir des valeurs échantillonnées desdits au moins quatre signaux successifs, de calculer la température au niveau de l'applicateur, indépendamment du coefficient de réflexion de l'applicateur vis-à-vis de la zone à traiter et des pertes des commutateurs micro-onde et des câbles d' interconnexion.

3. Système selon l'une des revendications 1 ou 2, caractérisé en ce que, dans le cas du traitement d'un tissu vivant tel que la prostate, celui-ci comporte :

- une première (20), une deuxième (21) et une troisième (28) voies de transmission connectées en parallèle en sortie dudit générateur d'énergie micro-onde (1),
- un premier (30) et un deuxième (31) applicateur micro- onde, le premier applicateur micro-onde, applicateur rectal, étant relié, d'une part, directement à la sortie de la première voie et, d'autre part, sélectivement à la sortie de la deuxième voie ou à l'entrée de mesure du radiomètre à deux références par l'intermédiaire d'un premier et d'un deuxième commutateur micro-onde multivoies, le deuxième applicateur, applicateur urétral, étant sélectivement relié, d'une part, à la sortie de la troisième voie ou, d'autre part, à l'entrée de mesure du radiomètre à deux références par l'intermédiaire dudit premier commutateur multivoies et d'un deuxième commutateur multivoies.

4. Système selon la revendication 3, caractérisé en ce que lesdits premier et deuxième applicateurs (30, 31) sont thermostatés.

5. Système selon l'une des revendications 3 et 4, caractérisé en ce que l'applicateur rectal (30) est susceptible d'être alimenté en énergie micro-onde par une voie déphasée et par une voie non déphasée de l'onde porteuse de l'énergie micro-onde et en ce que l'applicateur urétral (31) est alimenté en énergie micro-onde par une voie déphasée de l'onde porteuse de l'énergie micro-onde, la zone (Z) de la prostate à traiter étant située en cours de traitement entre lesdits deux applicateurs (30 et 31).

6. Système selon l'une des revendications 3 à 5, caractérisé en ce que lesdites deuxième (21) et troisième (22) voies comportent chacune un circuit déphaseur programmable (210, 211) de l'onde porteuse de l'énergie micro-onde transmise vers les applicateurs correspondants, permettant de moduler la phase relative de l'onde porteuse de l'énergie micro-onde transmise par deux voies consécutives selon un programme de modulation déterminé pour moduler le niveau d'énergie transféré au niveau de la zone (Z) à traiter ou d'une partie de celle-ci et d'atteindre pour cette dernière une température d'asservissement.

7. Système selon la revendication 6, caractérisé en ce que la modulation programmée de la phase relative de l'onde porteuse de l'énergie micro-onde est effectuée continûment ou par incréments discrets.

8. Système selon l'une des revendications 6 et 7, caractérisé en ce que la pente (p) ou loi de convergence vers la température d'asservissement est choisie de façon à correspondre à une fonction monotone croissante de la température en fonction du temps, par incrément de température compris entre 0,8° C et 1,2° C par minute.

9. Système selon l'une des revendications 6 à 8, caractérisé en ce que la modulation de la phase relative de l'onde porteuse de l'énergie micro-onde transmise par deux voies successives est effectuée soit de façon continue par phase variable entre 0 degrés et 180 degrés, soit avec des valeurs fixées de déphasage.

10. Système selon l'une des revendications 3 à 9, caractérisé en ce que ledit deuxième applicateur urétral (31) comprend, dans une sonde de type sonde de Foley,

   - une antenne filaire (311) connectée au commutateur multivoies correspondant par un connecteur micro-onde et un câble coaxial,
   - un tuyau d'entrée d'eau (312) et un tuyau de sortie d'eau (313) dans la sonde, permettant d'assurer une circulation d'eau.

11. Système selon la revendication 10, caractérisé en ce que l'antenne filaire (311) comporte une butée (3113) disposée à une distance déterminée de l'extrémité de l'antenne filaire et permettant de régler la longueur effective de l'antenne filaire dans la sonde de type Foley.

12. Système selon la revendication 10 ou 11, caractérisé en ce que ladite antenne filaire (311) est formée par ledit câble coaxial présentant successivement à son extrémité dans le sens de propagation direct de l'énergie micro-onde :

   - une partie (3111) de longueur h sur laquelle le blindage du câble coaxial a été supprimé, l'âme centrale dudit câble coaxial étant, sur cette partie, recouverte par le matériau diélectrique du câble,
   - une partie (3112) de longueur h' formée par ladite âme centrale du câble coaxial totalement dénudé, les longueurs h et h' étant déterminées en fonction de la fréquence f0 de l'énergie micro-onde.

13. Système selon la revendication 12, caractérisé en ce que, en vue d'applications en gynécologie, les parties de longueur h et h' sont recouvertes d'une coiffe en matériau diélectrique, permettant d'assurer une adaptation d'impédance de l'antenne filaire.

14. Procédé de calibration ou d'étalonnage d'une système pour le traitement interne de corps certains, selon l'une des revendications 1 à 13, caractérisé en ce qu'il consiste, préalablement et/ou au cours du processus de traitement proprement dit:

   a)

   - à connecter l'entrée de mesure du radiomètre (4) à deux références à la première sortie (421) du module d'étalonnage (420), ladite entrée de mesure dudit radiomètre à deux références étant ainsi placée en court-circuit,
   - à connecter le deuxième commutateur multivoies (49) du radiomètre à deux références sur la première TR1 puis sur la deuxième TR2 référence de température,
   - à mesurer et mémoriser les valeurs correspondantes Vo1, Vo2, du signal délivré par le circuit d'échantillonnage,

   b)

   - à connecter l'entrée de mesure dudit radiomètre à deux références à la deuxième sortie (422) dudit module d'étalonnage, ladite entrée de mesure étant connectée à la résistance de valeur déterminée portée à la température T1 de valeur déterminée,
   - à connecter le deuxième commutateur multivoies du radiomètre à deux références sur la première TR1 puis sur la deuxième TR2 référence de température,
   - à mesurer et mémoriser les valeurs V11, puis V12, correspondantes du signal délivré par le circuit d'échantillonage,

   c)

   - à connecter l'entrée de mesure dudit radiomètre à deux références à la troisième sortie (423) dudit module d'étalonnage, ladite entrée de mesure étant connectée à la résistance de valeur déterminée portée à la température T2 de valeur déterminée,

- à connecter le deuxième commutateur multivoies dudit radiomètre à deux références sur la première TR1 puis sur la deuxième TR2 référence de température,
- à mesurer et mémoriser les valeurs V21, V22, correspondantes du signal délivré par le circuit d'échantillonage (416),

d)

- à résoudre le système d'équations linéaires liant les valeurs mémorisées Vo1, Vo2 ; V11, V12 ; V21, V22, lesdites valeurs vérifiant chacune une relation de la forme

$$Vij = K.\Delta F (a.K2 \times T \times (1-\rho) + Tcab.K2(1-a).(1-\rho) - a^2\rho + TRj(a^2+K^2 2.\rho-K^2 1)$$

où

| | |
|---|---|
| Vij | désigne chaque valeur mémorisée précitée, |
| K | désigne le produit du gain G de la chaîne, par la constante de Boltzman KB, |
| ΔF | désigne la bande passante du radiomètre à deux références, |
| TRj | désigne soit la première TR1, soit la deuxième TR2, température de référence, |
| K1 et K2 | désignent les coefficients de perte du premier commutateur micro-onde du radiomètre à deux références pour les voies 1 et 2 considérées, |
| a | désigne les pertes du câble permettant d'assurer la liaison entre entrée de mesure du radiomètre à deux références et des différentes entrées du module de calibration ou d'étalonnage, |
| Tcab | désigne la température du câble de liaison, avec $\rho = 0$, lors de l'interconnexion de l'entrée du radiomètre sur la deuxième ou troisième entrée et sur la résistance correspondante de valeur déterminée du module d'étalonnage et $\rho = 1$ lors de l'interconnexion de l'entrée du radiomètre sur la première entrée et sur le court-circuit du module d'étalonnage ou de calibration. |

15. Procédé selon la revendication 14, caractérisée en ce que les deux températures T1 et T2, auxquelles sont portées les résistances du module de calibration ou d'étalonnage (420) étant ajustables, ledit procédé de calibration consiste :

a') à effectuer l'étape a) précédente,
b') à effectuer les étapes b) ou c) précédentes relativement aux températures de référence TR1, successivement TR2, la température ajustable T1 ou T2 étant ajustée progressivement de façon à obtenir une valeur nulle pour les valeurs correspondantes V11, V12 ou V21, V22 délivrées par le circuit d'échantillonnage pour des valeurs correspondantes, dites températures de zéro, TRZ1, TRZ2, des températures ajustables T1, respectivement T2, chaque température de zéro vérifiant une relation du type :
$$TRZj = ( TRj.K1^2 - Tcab.K2(1-a) + Voj )/(a.K2.K.\Delta F) , où$$

j désigne l'indice pouvant prendre la valeur 1 ou 2,
K = G.KB .

## Claims

1. System for internally treating bodies by thermal means using microwave energy over an area to be treated, this system comprising

- a generator (1) of microwave energy at a first predetermined frequency, at least two paths (20, 21; 20, 21, 22) for transmitting the microwave energy emitted by the generator (1), each path generating a microwave treatment signal modulated in amplitude according to a predetermined periodic modulation law to a second predetermined frequency, the microwave energy carrier wave of two successive paths having a phase shift of predetermined value,

- at least one microwave applicator (30, 31) associated with each of said at least two paths to enable the microwave energy supplied by said paths to be applied to distinct points in the vicinity of the area (Z) to be treated and

- means (7) for calculating and displaying the radiated microwave energies and the instantaneous temperatures of the distinct points of the area to be treated,

- a radiometer (4) with two internal temperature references selectively interconnected to said at least one microwave applicator per path acting as a sensor and measuring the absolute temperature of the corresponding distinct point in the vicinity of the area to be treated by the application of microwave energy, characterized in that it also comprises,

- a calibration module (420) with three outputs connected to said radiometer (4) with two internal references,

- a first output (421) of the calibration module being connected to an electric short circuit in the microwave frequency band of the radiometer,

- a second output (422) of the calibration module being connected to a first calibrated resistor of predetermined value in the frequency band of the radiometer, this resistor being brought to a first temperature T1 of predetermined value in operation,

- a third output (423) of the calibration module being connected to a second calibrated resistor of the same predetermined value in the frequency band of the radiometer, this second resistor being brought to a predetermined temperature T2, in operation,

- a multipath microwave switch (643) allowing either one of the microwave applicators (30, 31) associated with said paths or one of the outputs of said calibration module (420) to be connected selectively to the input of the microwave switch forming a measuring input of said radiometer with two references.

2. System according to claim 1, characterized in that said radiometer with two references comprises:

- a first microwave switch (43) comprising two connected input paths, one (43a) to the output of an applicator (30, 31) by means of a multipath switch and the other (43b) to the microwave short-circuit and an output (43c) connectable to one or other of the two inputs, a circulator (411) with three successive paths, the first (411a) being connected to the output of the first microwave switch and the second (411b) to the input of an electronic microwave treatment unit (414, 415, 416, 417), the third path (411c) constituting a reference input,

- at least two internal temperature reference sources (47, 48) and one second microwave switch (49) with at least two input paths each connected to the output of said at least two reference sources (47, 48) and an output connected to said third reference input of the circulator (411), said electronic treatment unit comprising:

- a synchronization control circuit (417) acting on both the first mircowave switch and the second microwave switch to obtain at least four different successive output signals, each of said at least four signals corresponding to each of the at least four possibilities for connection of the two microwave switches, the first with two input paths and the second with at least two input paths,

- a microwave detector (415) with one input connected by means of a microwave amplifier (414) to the output of the circulator,

- a calibration circuit (416) connected at the output of the microwave detector and supplying calibrated values of the signals supplied by the microwave detector to said calculation and display means, said calculation means calculating, under the control of the control and synchronizing circuit, on the basis of the calibrated values of said at least four successive signals, the temperature in the region of the applicator, independently of the coefficient of reflection of the applicator relative to the area to be treated and the losses from the microwave switches and interconnecting cables.

3. System according to one of claims 1 or 2, characterized in that, when treating a living tissue such as the prostate, it comprises:

- a first (20), a second (21) and a third (28) transmission path connected in parallel at the output of said microwave energy generator (1),

- a first (30) and a second (31) microwave applicator, the first microwave applicator, a rectal applicator, being connected, on the one hand, directly to the output of the first path and, on the other hand, selectively to the output of the second path or to the measurement input of the radiometer with two references by means of a first and a second multipath microwave switch, the second applicator, a urethral applicator, being selectively connected, on the one hand, to the output of the third path or, on the other hand, to the measurement input of the radiometer with two references by means of said first multipath switch and a second multipath switch.

4. System according to claim 3, characterized in that said first and second applicators (30, 31) are thermostatically controlled.

5. System according to one of claims 3 and 4, characterized in that the rectal applicator (30) can be supplied with microwave energy via a phase-shifted path and via a non-phase-shifted path of the microwave energy carrier wave and in that the urethral applicator (31) is supplied with microwave energy via a phase-shifted path of the microwave energy carrier wave, the area (Z) of the prostate to be treated being situated between said two applicators (30 and 31) during treatment.

6. System according to one of claims 3 to 5, characterized in that said second (21) and third (22) paths each comprise a programmable phase-shifting circuit (210, 211) of the microwave energy carrier wave transmitted to the corresponding applicators, allowing the relative phase of the microwave energy carrier wave transmitted by two successive paths to be modulated according to a predetermined modulation programme to modulate the energy level transferred to the level of the area (Z) to be treated or of a portion thereof and to attain an automatic control temperature for it.

7. System according to claim 6, characterized in that the programmed modulation of the relative phase of the microwave energy carrier wave is effected continuously or by discrete increments.

8. System according to one of claims 6 and 7, characterized in that the slope (p) or law of convergence to the automatic control temperature is selected so as to correspond to an increasing monotone function of the temperature as a function of time, by temperature increments between 0.8°C and 1.2°C per minute.

9. System according to one of claims 6 to 8, characterized in that the modulation of the relative phase of the microwave energy carrier wave transmitted by two successive paths is effected either continuously by variable phase between 0° and 180° or with fixed phase-shift values.

10. System according to one of claims 3 to 9, characterized in that said second urethral applicator (31) comprises, in a probe of the Foley probe type,

   - a wire antenna (311) connected to the corresponding multipath switch by a microwave connector and a coaxial cable,

   - a water inlet pipe (312) and a water outlet pipe (313) in the probe allowing the circulation of water.

11. System according to claim 10, characterized in that the wire antenna (311) comprises an abutment (3113) arranged at a predetermined distance from the end of the wire antenna and enabling the effective length of the wire antenna in the Foley type probe to be adjusted.

12. System according to claim 10 or 11, characterized in that said wire antenna (311) is formed by said coaxial cable having successively at its end in the direction of direct propagation of the microwave energy:

   - a portion (3111) of length h on which the cladding of the coaxial cable has been removed, the central core of said coaxial cable being covered by the dielectric material of the cable over this portion,

   - a portion (3112) of length h' formed by said central core of the completely bare coaxial cable, the lengths h and h' being determined as a function of the frequency f0 of the microwave energy.

13. System according to claim 12, characterized in that, for gynaecological applications, the portions of length h and h' are covered by a cap made of dielectric material allowing the impedance of the wire antenna to be adapted.

**14.** Process for gauging or calibrating a system for the internal treatment of bodies, according to one of claims 1 to 13, characterized in that it involves, prior to and/or during the actual treatment process:

a) connecting the measurement input of the radiometer (4) with two references to the first output (421) of the calibration module (420), said measurement input of said radiometer with two references thus being placed in a short-circuit,

- connecting the second multipath switch (49) of the radiometer with two references to the first TR1 then to the second TR2 temperature reference,

- measuring and storing the corresponding values Vo1, Vo2 of the signal supplied by the calibration circuit,

b) connecting the measurement input of said radiometer with two references to the second output (422) of said calibration module, said measurement input being connected to the resistor of predetermined value brought to the temperature T1 of predetermined value,

- connecting the second multipath switch of the radiometer with two references to the first TR1 then to the second TR2 temperature reference,

- measuring and storing the corresponding values V11 then V12, of the signal supplied by the calibration circuit.

c) connecting the measurement input of said radiometer with two references to the third output (423) of said calibration module, said measurement input being connected to the resistor of predetermined value brought to the temperature T2 of predetermined value,

- connecting the second multipath switch of said radiometer with two references to the first TR1 then to the second TR2 temperature reference,

- measuring and storing the corresponding values V21, V22 of the signal supplied by the calibration circuit (416),

d) resolving the system of linear equations linking the stored values Vo1, Vo2; V11, V12; V21, V22, said values each verifying a relationship of the type

$$Vij = K.\Delta F (a.K2 \times T \times (1-\rho) + Tcab.K2(1-a).(1-\rho) - a^2\rho + TRj(a^2+K^2 2.\rho-K^2 1)$$

wherein

| | |
|---|---|
| Vij | designates each aforementioned stored value, |
| K | designates the product of the gain G of the chain by the Boltzman constant KB, |
| $\Delta F$ | designates the passing band of the radiometer with two references, |
| TRj | designates either the first TR1 or the second TR2 reference temperature, |
| K1 and K2 | designate the coefficients of loss of the first microwave switch of the radiometer with two references for the paths 1 and 2 under consideration, |
| a | designates the losses of the cable connecting the measurement input of the radiometer with two references and the various inputs of the gauging and calibration module, |
| Tcab | designates the temperature of the connecting cable wherein $\rho = 0$ when the input of the radiometer is interconnected with the second or third input and with the corresponding resistor of predetermined value of the calibration module and $\rho = 1$ when the radiometer input is interconnected with the first input and the short-circuit of the calibration or gauging module. |

**15.** Process according to claim 14, characterized in that, as the two temperatures T1 and T2 to which the resistors of the gauging or calibration module are brought are adjustable, said gauging process involves:

a') carrying out the foregoing stage a),

b') carrying out the foregoing stages b) or c) relating to the reference temperatures TR1 and TR2 successively, the adjustable temperature T1 or T2 being adjusted progressively so as to obtain a zero value for the corresponding values V11, V12 or V21, V22 supplied by the calibration circuit for corresponding values, known as zero temperatures, TRZ1, TRZ2, of the adjustable temperatures T1 and T2 respectively, each zero temperature verifying a relationship of the type:

$$TRZj = (TRj.K1^2 - Tcab.K2(1-a) + Voj)/(a.K2.K\Delta F) \text{, wherein}$$

j designates the index which can have a value of 1 or 2,
K = G.KB

**Patentansprüche**

1.  System für die innere Wärmebehandlung von bestimmten Körpern durch Aufbringung von Mikrowellenenergie auf eine zu behandelnde Zone, wobei dieses System aufweist

    -   einen Generator (1) für Mikrowellenenergie von einer ersten bestimmten Frequenz, wenigstens zwei Übertragungskanälen (20, 21; 20, 21, 22) für die Mikrowellenenergie, die durch den Generator (1) emittiert wird, wobei jeder Kanal es erlaubt, ein Mikrowellenbehandlungssignal zu erzeugen, das in der Amplitude gemäß einem bestimmten periodischen Modulationsgesetz in eine zweite bestimmte Frequenz moduliert ist, wobei die Trägerwellen der Mikrowellenenergie von zwei aufeinanderfolgenden Kanälen eine vorgegebene Phasenverschiebung aufweisen,

    -   wenigstens eine Mikrowellenaufbringungseinrichtung (30, 31), die jedem der wenigstens zwei Kanäle zugeordnet ist und es erlaubt, die Aufbringung von Mikrowellenenergie, welche durch die Kanäle zugeführt worden ist, auf bestimmte Punkte in der Nachbarschaft der zu behandelnden Zone (Z) zu gewährleisten,

    -   Mitteln (7) zur Berechnung und Anzeige der ausgestrahlten Mikrowellenleistungen und der momentanen Temperaturen der bestimmten Punkte der zu behandelnden Zone,

    -   ein Radiometer (4) für zwei innere Referenztemperaturen, das wahlweise mit der wenigstens einen Mikrowellenaufbringungseinrichtung pro Kanal verbunden ist, die Rolle eines Aufnehmers spielt und es erlaubt, die absolute Temperatur des entsprechenden bestimmten Punktes in der Umgebung der zu behandelnden Zone durch die Aufbringung von Mikrowellenenergie zu messen, **dadurch gekennzeichnet**, daß es auch aufweist

    -   ein Eichmodul (420) mit drei Ausgängen, das mit dem Radiometer (4) für zwei innere Referenzen verbunden ist,

    -   wobei ein erster Ausgang (421) des Eichmoduls mit einem elektrischen Kurzschluß in dem Mikrowellenfrequenzband des Radiometers verbunden ist,

    -   wobei ein zweiter Ausgang (422) des Eichmoduls mit einem ersten Widerstand, der auf einen bestimmten Wert in dem Frequenzband des Radiometers kalibriert ist, verbunden ist, wobei der Widerstand auf einer ersten Temperatur T1 bestimmten Wertes im Betrieb gehalten wird,

    -   wobei ein dritter Ausgang (4, 23) des Eichmoduls mit einem zweiten Widerstand verbunden ist, der auf denselben bestimmten Wert in dem Frequenzband des Radiometers kalibriert ist, wobei der zweite Widerstand im Betrieb auf einer bestimmten Temperatur T2 gehalten wird,

    -   einen Mehrkanal-Mikrowellenwandler (643), der es erlaubt, wahlweise eine der Mikrowellenaufbringungseinrichtungen (30, 31), die den Kanälen zugeordnet sind, oder einen der Ausgänge des Eichmoduls (420) an den Eingang des Mikrowellenwandlers, der den Meßeingang des Radiometers für zwei Referenzen bildet, anzuschließen.

2.  System gemäß dem Anspruch 1, **dadurch gekennzeichnet**, daß das Radiometer für zwei Referenzen aufweist:

    -   einen ersten Mikrowellenwandler (43), der zwei Eingangskanäle, von denen der eine (43a) an den Ausgang einer Aufbringungseinrichtung (30, 31) unter der Zwischenschaltung eines Mehrkanal-Wandlers und der andere (43b) an den Mikrowellenkurzschluß angeschlossen ist, und einen Ausgang (43c), der an den einen oder den anderen der beiden Eingänge anschließbar ist, eine Richtungsgabel (411) mit drei aufeinanderfolgenden Kanälen, von denen der erste (411a) an den Ausgang des ersten Mikrowellenwandlers und der zweite (411b) an den Eingang einer elektronischen Mikrowellenbehandlungsanordnung (414, 415, 416, 417) angeschlossen ist, wobei der dritte Kanal (411c) einen Referenzeingang bildet, aufweist,

- wenigstens zwei Quellen (47, 48) für innere Bezugstemperaturen und einen zweiten Mikrowellenwandler (49) mit wenigstens zwei Eingangskanälen, die jeweils an einen Ausgang der wenigstens beiden Bezugsquellen (47, 48) angeschlossen sind, und einem Ausgang, der mit dem dritten Bezugseingang der Richtungsgabel (411) verbunden ist, wobei die elektronische Behandlungsanordnung aufweist:

- eine Synchronisationsteuerschaltung (417), die sowohl auf den ersten Mikrowellenwandler als auch auf den zweiten Mikrowellenwandler einwirkt, um wenigstens vier aufeinanderfolgende unterschiedliche Ausgangssignale zu erhalten, wobei jedes der wenigstens vier Signale einer der wenigstens vier Möglichkeiten der Verbindung der beiden Mikrowellenwandler, der erste mit zwei Eingangskanälen und der zweite mit wenigstens zwei Eingangskanälen, entspricht,

- eine Mikrowellenerfassungseinrichtung (415) mit einem Eingang, der unter Zwischenschaltung eines Mikrowellenverstärkers (414) an den Ausgang der Richtungsgabel angeschlossen ist,

- eine Signalabtast-Schaltung (416), die mit dem Ausgang der Mikrowellenerfassungseinrichtung verbunden ist und abgetastete Werte der durch die Mikrowellenerfassungseinrichtung gelieferten Signale den Rechen- und Anzeigemitteln zuführt, wobei die Rechenmittel unter der Steuerung der Steuerungs- und Synchronisationsschaltung es erlauben, ausgehend von den erfaßten Werten der wenigsten vier aufeinanderfolgenden Signale die Temperatur auf der Höhe der Aufbringungseinrichtung unabhängig von dem Reflexionskoeffizienten der Aufbringungseinrichtung gegenüber der zu behandelnden Zone und von Verlusten der Mikrowellenwandler und der Verbindungskabel zu berechnen.

3. System nach einem der Ansprüche 1 oder 2, **dadurch gekennzeichnet**, daß es in dem Fall der Behandlung eines lebenden Gewebes wie der Prostata aufweist:

- einen ersten (20), einen zweiten (21) und einen dritten (23) Übertragungskanal, die parallel an den Ausgang des Mikrowellenenergiegenerators (1) angeschlossen sind,

- eine erste (30) und eine zweite (31) Mikrowellenaufbringungseinrichtung, wobei die erste Mikrowellenaufbringungseinrichtung, die rektale Aufbringungseinrichtung, einerseits direkt mit dem Ausgang des ersten Kanals und andererseits wahlweise mit dem Ausgang des zweiten Kanals oder dem Meßeingang des Radiometers für zwei Referenzen unter Zwischenschaltung eines ersten und eines zweiten Mehrkanal-Mikrowelllenwandlers angeschlossen ist, und wobei die zweite Aufbringungseinrichtung, die uretale Aufbringungseinrichtung, wahlweise einerseits mit dem Ausgang des dritten Kanals oder andererseits dem Meßeingang des Radiometers für zwei Referenzen unter Zwischenschaltung des ersten Mehrkanal-Wandlers und eines zweiten Mehrkanal-Wandlers angeschlossen ist.

4. System nach Anspruch 3, **dadurch gekennzeichnet**, daß die ersten und zweiten Aufbringungseinrichtungen (30, 31) thermostabilisiert sind.

5. System nach einem der Ansprüche 3 und 4, **dadurch gekennzeichnet**, daß die rektale Aufbringungseinrichtung (30) in der Lage ist, durch einen gegenüber der Trägerwelle der Mikrowellenenergie phasenverschobenen Kanal und einen gegenüber dieser nicht phasenverschobenen Kanal mit Mikrowellenenergie versorgt zu werden, und daß die uretale Aufbringungseinrichtung (31) durch einen gegenüber der Trägerwelle der Mikrowellenenergie phasenverschobenen Kanal mit Mikrowellenenergie versorgt wird, wobei die Zone Z der zu behandelnden Prostata während der Behandlung zwischen den beiden Aufbringungseinrichtungen (30, 31) angeordnet ist.

6. System nach einem der Ansprüche 3 bis 5, **dadurch gekennzeichnet**, daß die zweiten (21) und dritten (22) Kanäle jeweils eine programmierbare Phasenschieberschaltung (210, 211) der Trägerwelle der zu den entsprechenden Aufbringungseinrichtungen übertragenen Mikrowellenenergie aufweisen, die es erlauben, die Phase der durch die aufeinanderfolgenden beiden Kanäle übertragenen Mikrowellenenergie gegenüber der Trägerwelle gemäß einem bestimmten Modulationsprogramm zu modulieren, um das in den Bereich der zu behandelnden Zone Z oder eines Teils von dieser übertragene Energieniveau zu modulieren und für die letztere eine geregelte Temperatur zu erhalten.

7. System nach dem Anspruch 6, **dadurch gekennzeichnet**, daß die Programmierte Modulation der Phase gegenüber der Trägerwelle der Mikrowellenenergie kontinuierlich oder durch bestimmte Schritte bewirkt wird.

8. System nach einem der Ansprüche 6 und 7, **dadurch gekennzeichnet**, daß die Neigung (p) oder das Konvergenzgesetz zu der geregelten Temperatur in der Weise gewählt ist, daß sie einer monoton wachsenden Funktion der Temperatur als Funktion der Zeit entspricht, wobei die Temperaturanstiege zwischen 0,8° C und 1,2° C pro Minute verstanden werden.

9. System nach einem der Ansprüche 6 bis 8, **dadurch gekennzeichnet**, daß die Phasenmodulation gegenüber der Trägerwelle der durch die beiden aufeinanderfolgenden Kanäle übertragenen Mikrowellenenergie entweder kontinuierlich durch eine variable Phase zwischen 0° und 180° oder mit festen Phasenverschiebungswerten bewirkt wird.

10. System nach einem der Ansprüche 3 bis 9, **dadurch gekennzeichnet**, daß die zweite uretale Aufbringungseinrichtung (31) in einer Sonde der Foley-Sondenart umfaßt

   - eine fadenförmige Antenne (311), die mit dem entsprechenden Mehrkanal-Wandler durch eine Mikrowellenverbindungseinrichtung und ein Koaxialkabel verbunden ist,

   - einen Wassereinlaßschlauch (312) und einen Wasserauslaßschlauch (313) in der Sonde, die es erlauben, eine Wasserzirkulation zu gewährleisten.

11. System nach dem Anspruch 10, **dadurch gekennzeichnet**, daß die fadenförmige Antenne (311) einen Anschlag (311.3) aufweist, der mit einem vorgegebenen Abstand von dem Ende der fadenförmigen Antenne angeordnet ist und es erlaubt, die effektive Länge der fadenförmigen Antenne in der Sonde der Foley-Art einzustellen.

12. System nach dem Anspruch 10 oder 11, **dadurch gekennzeichnet**, daß die fadenförmige Antenne (311) durch das Koaxialkabel gebildet wird, das aufeinanderfolgend an seinem Ende in dem Sinn einer direkten Übertragung der Mikrowellenenergie aufweist:

   - einen Abschnitt (3111) der Länge h, in dem die Abschirmung des Koaxialkabels aufgehoben worden ist, wobei die zentrale Ader des Koaxialkabels in diesem Abschnitt durch das dielektrische Material des Kabels abgedeckt ist,

   - einen Abschnitt (312) der Länge h', der durch die zentrale Seele des Koaxialkabels, welche vollkommen entblößt worden ist, gebildet wird, wobei die Längen h und h' als Funktion der Frequenz fO der Mikrowellenenergie bestimmt sind.

13. System nach Anspruch 12, **dadurch gekennzeichnet**, daß im Hinblick auf gynäkologische Anwendungen die Abschnitte der Länge h und h' durch eine Abdeckung aus dielektrischem Material abgedeckt sind, die eine Adaptierung der Impedanz der fadenförmigen Antenne gewährleisten.

14. Verfahren der Kalibrierung und der Eichung eines Systems zur inneren Behandlung von bestimmten Körpern gemäß einem der Ansprüche 1 bis 13, **dadurch gekennzeichnet**, daß es vor/oder während des Vorgangs der Behandlung umfaßt:

   a)

   - daß der Meßeingang des Radiometers (4) für zwei Referenzen an den ersten Ausgang (421) des Eichmoduls (420) angeschlossen wird, wodurch der Meßeingang des Radiometers für zwei Referenzen in Kurzschluß gebracht wird,

   - daß der zweite Mehrkanal-Wandler (49) des Radiometers für zwei Referenzen auf die erste TR1 und dann auf die zweite TR2 Bezugstemperatur eingestellt wird,

   - daß die entsprechenden Werte Vo1, Vo2 des von der Signal-Abtastschaltung gelieferten Signals gemessen und gespeichert wird,

b)

- daß der Meßeingang des Radiometers für zwei Referenzen mit dem zweiten Ausgang (422) des Eichmoduls verbunden wird, wobei der Meßeingang mit dem Widerstand vorgegebenen Wertes verbunden wird, der auf einer Temperatur T1 vorgegebenen Wertes gehalten ist,

- daß der zweite Mehrkanal-Wandler des Radiometers für zwei Referenzen auf die erste TR1 und dann auf die zweite TR2 Bezugstemperatur eingestellt wird,

- daß die entsprechenden Werte V11 und dann V12 des von der Signal-Abtast-Schaltung gelieferten Signals gemessen und gespeichert werden,

c

- daß der Meßeingang des Radiometers für zwei Referenzen mit dem dritten Ausgang (423) des Eichmoduls verbunden wird, wobei der Meßeingang mit dem Widerstand vorgegebenen Wertes, welcher auf der Temperatur T2 vorgegebenen Wertes gehalten wird, verbunden ist,

- daß der zweite Mehrkanal-Wandler des Radiometers für zwei Referenzen erst auf die erste TR1 und dann auf die zweite TR2 Bezugstemperatur eingestellt wird,

- daß die entsprechenden Werte V21, V22 des von der Signal-Abtast-Schaltung (416) gelieferten Signals gemessen und gespeichert werden,

d)

- daß das System aus linearen Gleichungen, das die gespeicherten Werte Vo1, Vo2; V11, V12; V21, V22 verbindet, gelöst wird, wobei die Werte jeweils eine Relation der Form verifzieren

$$V_{ij} = K.\Delta F \, (a.K2 \times T \times (1-\rho) + Tcab.K2(1-a).(1-\rho) - a^2\rho + TRj(a^2+K22.\ \rho-K^21))$$

wobei

| | |
|---|---|
| Vij | den vorgenannten gespeicherten Wert bezeichnet, |
| K | das Produkt der Leistung G der Kette mit der Boltzmannkonstante KB bezeichnet, |
| $\Delta F$ | den Durchlaßbereich des Radiometers für zwei Referenzen bezeichnet, |
| TRj | sowohl die erste TR1 als auch die zweite TR2 Bezugstemperatur bezeichnet, |
| K1 und K2 | die Verlustkoeffizienten des ersten Mikrowellen-Wandlers des Radiometers für zwei Referenzen für die betrachteten Kanäle 1 und 2 bezeichnen, |
| a | die Verluste des Kabels bezeichnet, was es erlaubt, die Verbindung zwischen dem Meßeingang des Radiometers für zwei Referenzen und den unterschiedlichen Eingängen des Kalibrierungs- oder Eichmoduls herzustellen, |
| Tcab | die Temperatur des Verbindungskabels mit $\rho$ = 0 bei der Verbindung des Eingangs des Radiometers mit dem zweiten oder dritten Eingang und dem entsprechenden Widerstand bestimmten Wertes des Eichmoduls und mit $\rho$ = 1 bei einer Verbindung des Eingangs des Radiometers an den ersten Eingang und an den Kurzschluß des Eich- oder Kalibrierungsmoduls bezeichnet. |

15. Verfahren nach Anspruch 14, **dadurch gekennzeichnet**, daß die beiden Temperaturen T1 und T2, auf denen die Widerstände des Kalibrierungs- oder Eichmoduls (420) gehalten werden, einstellbar sind, wobei der Kalibrierungsvorgang umfaßt:

a') daß der vorher genannte Abschnitt a) ausgeführt wird,
b') daß die vorherigen Abschnitte b) oder c) bezüglich der Bezugstemperaturen TR1 und dann TR2 ausgeführt werden, wobei die einstellbare Temperatur T1 oder T2 progressiv in der Weise eingestellt wird, daß ein Wert 0 für die entsprechenden Werte V11, V12 oder V21, V22, welche von der Signal-Abtast-Schaltung für die entsprechenden Werte geliefert werden, erhalten werden, wobei die 0-Temperaturen TRZ1, TRZ2, die einstellbaren Temperaturen T1 bzw. T2 einer Relation der Art genügen:

$$TRZj = ( TRj.K1^2 - Tcab.K2(1-a) + Voj )/(a.K2.K\Delta F) \text{, wobei}$$

j den Indice bezeichnet, der den Wert 1 oder 2 annehmen kann,
K = G.KB

# FIG.1.

210 21

AMPLIFICATEUR MODULATEUR

5c 5

CORPS CERTAIN

31

Z

5d

5b

ADAPTATEUR

30

20

AMPLIFICATEUR MODULATEUR

5a

CIRCULATION DU FLUIDE

6

BLOC THERMOSTA-TISATION 8

4

RADIOMETRE

COMMANDE COMMUTATION

MESURE DES TEMPERATURES

7

COMMANDE EMISSION fo,f1

COMMANDE DEPHASAGE

MICROCALCULATEUR DE GESTION DES PHASES ET DES PUISSANCES ET DES MESURES DE TEMPERATURE

# FIG.2.

47  48

417a

417b

417c  417

42

49a  49d  49b  49

46

49c

43

413

414

415b  415

416d

43b

411c

44

415

416

43d

43c

412

415a

416

43a

411a  411

411b

427c

31

45

418

Z

VERS 7

FIG.3a.

FIG.3b.

FIG.3c.

FIG.3d.

# FIG.4.

DEPHASEURS 22 221

RELAIS MICROONDES

220

AMPLIFICATEUR

APPLICATEUR URETAL
THERMOSTATE

5

31

210 21 211

AMPLIFICATEUR

APPLICATEUR RECTAL
THERMOSTATE

30

1

CIRCULATION DU FLUIDE

8

AMPLIFICATEUR

20 201

BLOC THERMOSTATISATION

6

COMMANDE
COMMUTATION

RADIOMETRE A
DEUX REFERENCES

4

MESURE DES
TEMPERATURES

7

FIG.5a.

313

311

312

31

SONDE TYPE DE FOLEY

FIG.5b.

3110   3113   311   3111   3112

h   k'

FIG.5c.

3110   3114

PARTIE ACTIVE

$ER = 2E \cos \varphi$

T E30+E31

ER

E30   $\varphi$   E31

b)

FIG.6.

T

TASS

P

c)

t

26

# FIG.6a.

PROSTATE

31

Z

30